(19) Europäisches Patentamt
European Patent Office
Office européen des brevets

(11) **EP 3 230 359 B1**

(12) **EUROPÄISCHE PATENTSCHRIFT**

(45) Veröffentlichungstag und Bekanntmachung des Hinweises auf die Patenterteilung:
**18.03.2026  Patentblatt 2026/12**

(21) Anmeldenummer: **15808147.1**

(22) Anmeldetag: **09.12.2015**

(51) Internationale Patentklassifikation (IPC):
**C08L 1/02** (2006.01)    **A61Q 19/00** (2006.01)
**C08B 1/00** (2006.01)    **D01F 1/02** (2006.01)
**D01F 1/10** (2006.01)    **D01F 2/00** (2006.01)

(52) Gemeinsame Patentklassifikation (CPC):
**C08L 1/02; C08B 1/003; D01F 1/02; D01F 1/10; D01F 2/00**

(86) Internationale Anmeldenummer:
**PCT/EP2015/079148**

(87) Internationale Veröffentlichungsnummer:
**WO 2016/091963 (16.06.2016 Gazette 2016/24)**

(54) **VERFAHREN ZUR HERSTELLUNG VON CELLULOSEFUNKTIONSFORMKÖRPERN MIT GEZIELTER FREISETZUNG VON WIRKSTOFFEN**

METHOD FOR PRODUCING SHAPED FUNCTIONAL CELLULOSE ARTICLES WITH TARGETED RELEASE OF ACTIVE INGREDIENTS

PROCÉDÉ DE PRODUCTION DE CORPS MOULÉS AYANT UN GROUPE FONCTIONNEL CELLULOSE AVEC LIBÉRATION CIBLÉE DE SUBSTANCES ACTIVES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.12.2014  DE 102014018139**

(43) Veröffentlichungstag der Anmeldung:
**18.10.2017  Patentblatt 2017/42**

(73) Patentinhaber: **Smartpolymer GmbH**
**07407 Rudolstadt (DE)**

(72) Erfinder:
• **BAUER, Ralf-Uwe**
**07407 Rudolstadt (DE)**
• **MEISTER, Frank**
**07407 Rudolstadt (DE)**
• **MOOZ, Michael**
**07422 Saalfelder Höhe (DE)**
• **KRIEG, Marcus**
**99423 Weimar (DE)**
• **RIEDE, Sabine**
**07407 Uhlstädt-Kirchhasel (DE)**

(74) Vertreter: **Plate, Jürgen**
**Patentanwalt Dr. Plate**
**Mühlstraße 9A**
**65597 Hünfelden (DE)**

(56) Entgegenhaltungen:
**DE-A1- 102007 054 702**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung von Celluloseformkörpern mit gezielter Freisetzung von Wirkstoffeinschlüssen. Bei den Wirkstoffen handelt es sich um feste oder flüssige lipophile Wirkstoffe oder um W/O-Emulsionen. Dieses Verfahren führt dazu, dass die Wirkstoffe während der Herstellung der cellulosischen Formkörper im Formkörper fest eingebunden werden, aber im Gebrauch die Wirkstofffreisetzung gesteuert werden kann.

Bereits in WO 2009 062 657 wird ein Verfahren für die Einarbeitung von unpolaren Substanzen wie beispielsweise Phasenwechselwerkstoffen (PCM) und/oder unpolaren Wirkstoffen in Celluloseformkörper beschrieben. Bei dem dort dargestellten Stand der Technik handelt es sich um ein Lyocell-Verfahren. Es wird eine Emulsion aus Cellulose in einem wässrigen Direktlösemittel und der unpolaren Substanz hergestellt. Zur Stabilisierung dieser Emulsion wird nanoskalige, hydrophob modifizierte pyrogene Kieselsäure eingearbeitet und/oder flächige und/oder längliche nanoskalige Partikel zugesetzt. Durch ein solches Verfahren wird eine sehr feste Einbindung der unpolaren Substanzen in die umgebende Cellulosematrix erreicht, durch die eine gesteuerte Freisetzung weitestgehend verhindert wird. Ein analoges Vorgehen ist in DE 10 2006 046 358 A1 beschrieben. Es wird keine Lehre für eine gezielte Freisetzungsrate von eingeschlossenen Wirkstoffen gegeben.

Auch die direkte Einarbeitung von Paraffin in schmelzbare Kunststoffmatrizes ist in der DE-PS 10 2010 007 497 A1 beschrieben. Hierbei wird die plastifizierte Mischung aus 40 bis 75 % Paraffin und 60 bis 25 % polymerer Trägerkomponente, die aus 5 bis 20 % eines thermoplastifizierbaren Polymers, aus 5 bis 20 % eines Styrol-Blockcopolymeren und aus 0 bis 20 % eines oder mehrerer Additive besteht, bei 130 bis 220 °C durch eine Düsenöffnung extrudiert und augenblicklich nach Verlassen der Düse auf eine Temperatur von 10 bis 80 °C abgeschreckt. Hauptnachteil bei einem solchen Vorgehen ist die geringe Formkörperfestigkeit, die enorme Dehnung bis mehr als 500 % sowie die Notwendigkeit einer Nachverstreckung um den Faktor 2 bis 12. Zudem ist dieses Verfahren für die Einarbeitung in Schmelzen nicht übertragbar auf Verfahren für das Arbeiten mit wässrigen Lösungen, die im Regelfall sehr viel niedrigere Viskositäten aufweisen.

Mehrkomponentenfasern mit reversiblen thermischen Eigenschaften der Outlast Technologies Inc., die auch aus Cellulose mittels Lösungsspinnverfahren gefertigt werden können, sind in den Schutzrechten US 7,244,497 B2 sowie WO 2005/017 247 A2 und in der Gebrauchsmusterschrift DE 20 2004 021 259 U1 beschrieben. Derartige Fasern werden erhalten durch die Verwendung von Einschlussstrukturen, im Normalfall Kapseln, die das PCM enthalten oder durch Kern/Mantel- oder Island-in-the-Sea-Strukturen, bei denen das PCM-Material fest von nicht-PCM-haltigem Material umschlossen ist. Ziel sind reversible thermische Eigenschaften, die erhalten werden durch eine feste Einbindung der PCM-Materialien in den Verbundkörper, sie sind damit ungeeignet für eine gezielte Freisetzung von Wirkstoffen.

Schließlich beschreibt die Patentschrift US 5,153,066 die Einbindung thermotroper Farbstoffe in eine Polymermatrix bei der die Farbstoffphase in innen und außen liegenden Schichten der schützenden Polymermatrix eingebettet ist. Auch hier erfolgt das erfinderische Handeln nur mit dem Ziel eine Freisetzung bzw. frühzeitige Zersetzung des Wirkstoffes durch äußere Einflüsse wirksam zu unterbinden. Darüber hinaus wird keine Lehre gegeben, wie solche stabilisierten Zusammensetzungen bzw. W/O-Emulsionen für die Faserherstellung aus einer Lösung mit anschließender Extrusion verwendet werden können, da diese Zusammensetzungen bzw. Emulsionen schon das Endprodukt darstellen.

Des Weiteren ist die Verwendung von modifizierten Kieselsäuren zur mechanischen Stabilisierung von keratinischen Substanzen/Emulsionen bzw. zur Stabilisierung von W/O-Emulsionen aus DE69600181 T2 oder DE 102004014704 A1 bekannt. Der Hauptgrund für deren Verwendung liegt in einer Verstärkung der Wechselwirkungen von unterschiedlichen Mischungskomponenten und hat damit keinen Bezug zu einer gesteuerten Freisetzung von Wirkstoffen. Zudem wird keinerlei Lehre zur Einbindung dieser Emulsionen in Fasern nach dem Lösungsspinnverfahren unter den dort vorherrschenden hohen Temperaturen und Scherkräften gegeben.

Aus den Arbeiten von T. Fornes et al. (T. Fornes, P. Yoon, D. Hutter, H. Keskkula und D. Paul: Polymer 2002, 43, 5915) ist die Verwendung von Schichtsilikaten mit interkalierten organischen Modifikatormolekülen zur Steuerung der Exfolierung in Polymermischungen oder -blends bekannt. Dabei kann bereits die Struktur der interkalierten Modifikatoren eine Reihe von strukturellen Veränderungen der Polymer- und/oder Blendstruktur verursachen (vgl. T. Fornes, D. Hutter und D. Paul: Macromolecules 2004, 37, 1793). Der Separationsgrad der einzelnen Silikaplättchen kann bei einem solchen Vorgehen sehr unterschiedlich sein. Während bei sehr geringer Separation Mikrokomposite dominieren, erreicht man durch Inklusion von Molekül(kett)en in die Galerien der Silikaplättchen interkalierte oder auch exfolierte Strukturen, wobei durch die vollständige Freisetzung einzelner Silikaplättchen signfikante Veränderung der Wechselwirkung an den Phasengrenzen der Polymerkomponenten resultieren.

Zudem konnte Hasegawa et al. (N. Hasegawa, H. Okamoto, M. Kato, A. Usuki und N. Sato: Polymer 2003, 44, 2933) nachweisen, dass sich u.U. auch un- oder wenig modifizierte Nanoschichtsilikate, beispielsweise $Na^+$ Montmorillonite (NaMMT) bereits mit Wasser(dampf) exfolieren lassen, wobei ähnliche Polymerstrukturen wie bei der Verwendung von modifizierten Schichtsilikaten resultieren sollen. Wasser bzw. Wasserdampf können zu einer Quellung der NaMMT führen, wobei nachfolgend, ähnlich wie bei organisch modifizierten Schichtsilikaten, Molekülketten in die vergrößerten Galerieabstände intrudieren können. N. Fedullo et al. (N. Fedullo, M. Sclavons, C. Bailly, J.-M. Lefebvre und J. Devaux: Macromol. Symp. 2006, 233, 235) konnten zudem zeigen, dass eingebundene PA 6-Molekülketten z.T. sehr auswaschresistent sind, und dass selbst eine mehrfache Extraktion mit Hexafluoroisopropanol (HFIP) keine vollständige Entfernung der Einschlüsse erreichen konnte. Also auch durch ein solches Handeln kann keine gezielte Steuerung des Freisetzungsverhaltens erreicht werden. Die Verwendung dieser Schichtsilikate zur Stabilisierung von lipohilen Substanzen oder gar W/O-Emulsionen während der Herstellung von Celluloseformkörpern nach dem Lyocellverfahren wurde nicht beschrieben. In der DE 10 2007 054 702 A1 ist schließlich ein Verfahren zur Herstellung von cellulosischen Formkörpern mit Einschlüssen einer unpolaren organischen Verbindung offenbart. Darin wird eine Emulsion der unpolaren organischen Verbindung in einer Cellulose-Lösung bereitet und durch Zugabe eines hydrophoben viskositätssteigernden Mittels stabilisiert. Der Emulsion werden dann nanoskalige, flächige, hydrophobierte Partikel zugesetzt, die die Einschlüsse der unpolaren organischen Verbindung umgeben und eine Suspension bilden. Die nanoskaligen, flächigen, hydrophobierten Partikel sind vorzugsweise modifizierte Schichtsilikate. Die Formkörper werden nach einem Trocken-Nass-Extrusionsverfahren hergestellt.

Ausgehend von dem aufgezeigten Stand der Technik lag der Erfindung die Aufgabe zugrunde, ein Verfahren zu entwickeln, mit dem lipophile Wirkstoffzusammensetzungen in festem oder geschmolzenem Zustand ohne die Verwendung von Einschlussstrukturen oder Trägerstoffen oder als W/O-Emulsionen während der Herstellung von cellulosischen Formkörpern in den cellulosischen Formkörper eingebunden werden können, so dass die Wirkstoffe fein verteilt im Formkörper vorliegen und während der Koagulation, welche oftmals in wässrigem Medium stattfindet, nicht ausgewaschen werden. Besondere Schwierigkeiten treten dadurch auf, dass beim Prozess der Formkörperherstellung aus Lösungen oft sehr hohe Scherkräfte wirken, die eine Phasenseparation bewirken können und dass die Koagulation bzw. die Entfernung des Lösemittels häufig in wässrigen Medien stattfindet, wo das Lösemittel ausgewaschen wird und die Gefahr besteht, dass die Wirkstoffzusammensetzungen auch mit ausgewaschen werden. Im Rahmen der textilen Anwendung der erfinderischen Celluloseformkörper sollen Wirkstoffe gesteuert freigesetzt und so die enthaltenen Substanzen einer beabsichtigten Anwendung zugeführt werden, ohne die im Stand der Technik dargestellten technischen und ökonomischen Nachteile in Kauf nehmen zu müssen. Zudem lag der Erfindung die Aufgabe zugrunde, auch lipophile, aber feuchtigkeitsaffine Wirkstoffe in gelöster oder dispergierter Form waschpermanent zu speichern und kontrolliert über einen längeren Zeitraum an die Formkörperumgebung abgeben zu können. Eine weitere erfinderische Aufgabe lag darin, die funktionalisierten Formkörper während der Gebrauchsphase mit stark flüchtigen oder thermo- bzw. chemosensiblen Wirkstoffen wieder beladen zu können.

Diese Aufgaben werden erfindungsgemäß durch ein mehrstufiges Verfahren gelöst, bei dem:

a) Zellstoff in einem wässrigen Direktlösungsmittel für Cellulose, wie NMMO, ionische Flüssigkeiten, oder ggf. Mischungen organischer Flüssigkeiten mit den genannten Direktlösemitteln, oder DMAc/-LiCl, dispergiert wird,

b) in einem separaten Verfahrensschritt ein organisch modifiziertes, oder ein durch Ionenaustausch mit Alkali- bzw. Erdalkali-Ionen höherer Perioden (z.B. $K^+$-, $Ca^{2+}$-, $Al^{3+}$-Ionen), oder ein durch Wasser voraktiviertes, nanoskaliges Schichtsilikat, mit einer wässrigen Lösung des Direktlösemittels in einem Ultraturrax homogenisiert und durch Einstellung von Scherrate (Drehzahl) und Scherdauer in einem definierten Grad partiell oder vollständig exfoliert wird, der Maische zugegeben und mit der Cellulosemaische gemischt wird,

c) in einem weiteren separaten Verfahrensschritt eine Zusammensetzung aus einem Wirkstoff und einem lipophilen Matrixmaterial für den Wirkstoff oder eine wirkstoffhaltige W/O-Emulsion durch anorganische oder organische Verdicker stabilisiert und in eine gelartige Paste überführt wird, diese Paste ebenfalls der Cellulosemaische zugegeben und unter Rühren bei lösungsmittelabhängigen Temperaturen bis 130 °C mit ihr vermischt wird,

d) aus der Mischung Wasser abgezogen wird bis eine vollständige Auflösung der Cellulose erfolgt ist, und

e) die entstandene Spinnlösung nach einem der bekannten Lösungsspinnverfahren zu Formkörpern wie beispielsweise Stapelfasern, Filamenten, Filmen oder Direktvliesen verformt, und gegebenenfalls nach einem der bekannten Prozesse nachbehandelt, gegebenenfalls aviviert und getrocknet wird.

Unter wirkstoffhaltiger lipophiler Substanz verstehen wir eine Mischung aus Wirkstoff und einem lipophilen Matrixmaterial. Wirkstoffhaltige W/O-Emulsionen gestatten es, neben lipophilen Wirkstoffen auch hydrophile Wirkstoffe, gelöst in Wasser oder hydrophilen Lösungsmitteln in eine lipophile Matrix zu "verpacken". Es gibt auch Prozesse, bei denen man eine W/O-Emulsion nochmals in eine lipophile Matrix "verpackt".

Bei den Lösungsspinnverfahren ist ein Lyocell-Spinnverfahren bevorzugt, es handelt sich also zumeist um einen ‚Trocken-Nass-Spinnprozess'.

Als nanoskalig werden im Zusammenhang mit der vorliegenden Erfindung Substanzen bzw. Schichtsilikate bezeichnet, die in mindestens einer Dimension eine Ausdehnung von 100 nm oder weniger aufweisen.

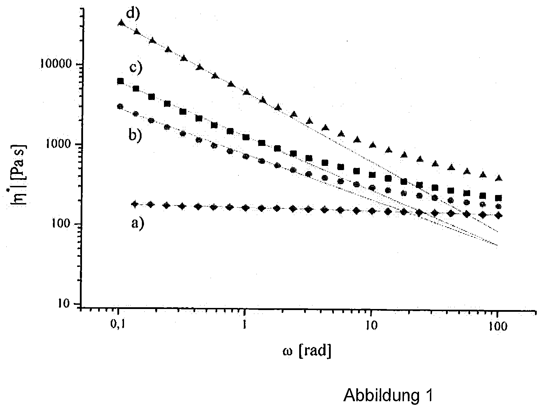

In Stufe b) wird das Schichtsilikat vorgequollen, das heißt, durch Interkalierung mit beispielsweise Ammoniumsalzen langkettiger Fettamine, Alkalimetall-, Erdalkalimetall- oder Borgruppen-Kationen höherer Perioden (3. Periode oder höher) des PSE, oder Wasser vergrößert sich der Abstand zwischen den einzelnen Lamellen (Schichten) des Schichtsilikates, was einen entscheidenden Einfluss auf den Grad der Exfolierung in der Spinnlösung hat. Gleichzeitig erhöht sich die Viskosität dieser Dispersion. Der Grad der Interkalierung wird beeinflusst durch die Größe der interkalierten Verbindungen, der Menge an interkalierten Wasser sowie durch eine definierte Einstellung von Scherdauer und Scherrate (vgl. Abbildung 1). Das Vermischen in der Stufe c) erfolgt bevorzugt in nicht mehr als 15 min, besonders bevorzugt für etwa 10 min.

Die anorganischen oder organischen Verdicker in Schritt c) umfassen Nanopartikel auf der stofflichen Grundlage von pyrogenen Kieselsäuren, Metalloxidkeramiken oder lösungsmittelverträgliche Metallnanopartikel und/oder aliphatisch-aromatische Blockcopolymere. Dabei können die aspektlosen Nanopartikel auf der stofflichen Grundlage von pyrogenen Kieselsäuren oder Metalloxidkeramiken organisch modifiziert sein.

Neben der Steuerung der Exfolierung der Schichtsilikate zur Kontrolle der Freisetzungsraten für die Wirkstoffe übernehmen die Schichtsilikate auch eine wichtige Rolle als Phasenvermittler im System.

Überraschenderweise wird dabei gefunden, dass insbesondere durch die separaten Schritte b) und c) die lipophilen Wirkstoffzusammensetzungen bzw. wirkstoffhaltigen W/O-Emulsionen stabil in die Spinnmasse eingebunden werden können, ohne dass es einer weiteren Stabilisierung der Mischungen oder Verkapselung der Wirkstoffe bedurfte. Sie liegen am Ende im Formkörper fein verteilt in Form von Domänen vor, worunter Bereiche verstanden werden, die funktional und strukturell (quasi-) unabhängig von benachbarten Abschnitten sind.

Ebenso erstaunlich, und für den Fachmann in keiner Weise vorherzusehen war, dass durch das Vorquellen des verwendeten Schichtsilikates im separaten Verfahrensschritt b) mehr noch als durch das Scheren des Gemisches aus Celluloselösung, Wirkstoffzusammensetzung und Schichtsilikat der Grad der Exfolierung der Schichtsilikate und damit die Freisetzung der eingebundenen Wirkstoffe bzw. W/O-Emulsion in Zeit und Intensität gesteuert werden kann.

Bei den Schichtsilikaten im Verfahrensschritt b) handelt es sich vorzugsweise um organisch modifizierte Schichtsilikate, die in den Galerien der Schichtsilikatplättchen organische Moleküle enthalten, die eine Anbindung an die Cellulose fördern und gleichzeitig die feine Verteilung der lipophilen Substanzen (Wirkstoffzusammensetzungen) bzw. W/O Emulsionen in der Cellulosematrix stabilisieren. Aber auch nicht modifizierte Schichtsilikate, wie beispielsweise Na-Montmorillonit, können dann eingesetzt werden, wenn hydrophilere Wirkstoffe oder besonders bevorzugt W/O Emulsionen in die Cellulosefasermatrix eingebunden und nachfolgend freigesetzt werden sollen. Es wurde gefunden, dass der Grad der Interkalierung/Exfolierung der Schichtsilikate einen entscheidenden Einfluss darauf ausübt, wie fest die lipophilen Substanzen bzw. Wirkstoffzusammensetzungen in der Cellulosematrix verankert sind. Dies lässt sich mit dem Aufbau der Schichtsilikate wie folgt erklären:

Bekanntermaßen sind Schichtsilikate aus parallel gestapelten Silikatplättchen (Einzellamellen) aufgebaut, wobei diese wiederum einen Drei-Lagen-Aufbau (ca. 1 nm Schichtdicke) aus alternierend tetraedrisch und oktaedrisch koordinierten Kationenschichten aufweisen, welche mit einer gemeinsamen Anionenschicht verbunden sind. In den interlamellaren Zwischenschichten (Galerien) sind zum Ladungsausgleich (isomorpher Kationenaustausch in den Kationenschichten) erforderliche, mobile Kationen angeordnet, die wiederum sehr einfach durch "organische Kationen", bevorzugt Ammonium- oder Phosphoniumkationen mit mindestens einem längeren, unverzweigten, gesättigten oder ungesättigten Kohlenwasserstoffrest, der 14 oder mehr Kohlenstoffatome, besonders bevorzugt 14 bis 20 C-Atome, speziell 14, 16 oder 18 C-Atome, aufweist ersetzt sein können. Als "ungesättigte Kohlenwasserstoffreste" werden insbesondere unverzweigte Alkylreste mit 1, 2 oder 3 Doppelbindungen bezeichnet. Insbesondere durch die Interkalierung mit den genannten organischen Kationen, die Alkyl- und/oder Alkenylgruppen aufweisen, werden die zwischenlamellaren Wechselwirkungen, d.h. die Kohäsion der Einzellamellen verändert. Die Alkyl- oder Alkenylgruppen können substituiert sein, insbesondere durch Hydroxy- oder Carboxygruppen. Dies wiederum wirkt sich auf eine mehr oder minder schnelle Aufspaltung in Einzellamellen (Exfolierung) aus. Gleiches wird bei nicht organisch modifizierten Schichtsilikaten erreicht, wenn beispielsweise die in den Galerien enthaltenen Kationen, z.B. Natriumionen, durch gleichwertige Kationen aus höheren Perioden des PSE, z.B. Kaliumionen ausgetauscht, oder die Schichtsilikate in polaren Lösungsmitteln wie beispielsweise Wasser vorgequollen werden.

Eine vollständige Exfolierung (Delaminierung), d.h. die vollständige Aufspaltung der Schichtsilikate in Silikatplättchen (Einzellamellen), führt zu einer festen Verankerung der lipophilen Substanzen bzw. Wirkstoffzusammensetzungen an den Einzellamellen und in der Cellulosematrix, während eine schwache Exfolierung/Interkalierung zu Formkörpern führt, die nach der Celluloseformkörperherstellung noch Wirkstoffe enthalten, diese aber in Abhängigkeit zum eingestellten Exfolierungsgrad relativ schnell wieder abgegeben werden.

Die Exfolierung kann insofern

- grundsätzlich durch die chemische Struktur und Konzentration der organischen Kationen im Schichtsilikat,

- durch die Größe der interkalierten Ionen oder den Quellgrad nicht organisch modifizierter Schichtsilikate,
- in den Schritten b) und c) durch die Temperatur, die Viskosität des umgebenden Mediums (verwendetes Dispergiermittel, Feuchtigkeit und in den stabilisierten Wirkstoffmischungen verwendete Kohlenwasserstoffe) sowie die Intensität und Dauer der Mischung/Scherung der Schichtsilikate und
- in den Schritten b) bis d) durch die rheologischen Eigenschaften des umgebenden Mediums (insofern auch die Vollständigkeit der Celluloseauflösung im Löseschritt), die Temperatur und ebenfalls die Scherintensität sowie -dauer beeinflusst werden.

Bei ausgewählten Wirkstoffen (z.B. Riechstoffe), die sich aufgrund zu starker Flüchtigkeit bzw. thermischer oder/und chemischer Sensibilität im Faserbildungs- und Faserverarbeitungsprozess entlang der textilen Wertschöpfung nicht direkt in den Cellulosefunktionsformkörper einbinden ließen, konnte zudem auch eine Beladung/Wiederbeladung der Funktionsformkörper mit Wirkstoffen in wirkungsrelevanten Mengen durchgeführt werden.

Für die Herstellung der erfinderischen, Wirkstoff freisetzenden Fasern können alle typischen Dissolvingzellstoffe wie Hart- und Weichholzzellstoffe mit hohen bis höchsten $\alpha$-Cellulosegehalten (> 80 %), Hochalphazellstoffe (Cotton Linters) sowie Zellstoffe von Einjahrespflanzen mit $\alpha$-Gehalten größer oder gleich 90 % genutzt werden.

Als erfindungsgemäße feste oder flüssige Wirkstoffzusammensetzungen können bevorzugt, aber nicht ausschließlich kosmetische Wirkstoffzusammensetzungen wie Nachtkerzen-, Johanneskraut-, Jojoba-, oder Avocadoöl, fettlösliche Vitamine und Provitamine, wie Vitamin A, Retinol, Vitamin D oder Vitamin E, wirkstoffhaltige W/O Emulsionen oder unpolare Pflanzenauszüge in Konzentrationen von 0,1 bis 200 g pro Kilogramm Cellulose eingesetzt werden.

Zu den bekannten Direktlösungsmitteln zählen beispielsweise NMMO, ionische Flüssigkeiten sowie ggf. Mischungen organischer Flüssigkeiten mit den genannten Direktlösemitteln oder DMAc/LiCl.

Als Schichtsilikate werden natürliche und organisch modifizierte Tonminerale wie beispielsweise Talk, Montmorillonit, Bentonit oder Kaolinit bzw. synthetische und organisch modifizierte Silikate wie beispielsweise Nanofil®, Laponite® oder Hectorit® verwendet. Der Gehalt an Schichtsilikat in der Cellulosefunktionsfaser beträgt 0,5 bis 20 %, bevorzugt 5 bis 15 % bezogen auf die eingesetzte Menge an Cellulose.

Zur Stabilisierung der hochviskosen Mischungen aus wirkstoffhaltiger lipophiler Substanz, gegebenenfalls in Kombination mit Kohlenwasserstoffen bzw. der W/O-Emulsionen in Verfahrensschritt 1 c) werden Nanopartikel auf der stofflichen Grundlage von pyrogenen Kieselsäuren, Metalloxidkeramiken oder lösungsmittelverträgliche Metallnanopartikel und/oder aliphatisch-aromatische Blockcopolymere in Konzentrationen von 0,1 bis 10 % bezogen auf die Gesamtmenge der Mischung aus Wirkstoff bzw. Wirkstoff/ Kohlenwasserstoff oder W/O-Emulsion genutzt.

Bei den W/O-Emulsionen handelt es sich um wässrige Zubereitungen von Kosmetikwirkstoffen, wie Harnstoff, oder um wässrige Auszüge von Pflanzeninhaltsstoffen jeweils gemischt mit unpolaren Kohlenwasserstoffen, Fettalkoholen, Fettsäuren und Fettsäureestern mit mehr als 8 Kohlenstoffatomen (im Fall der Fettsäureester mehr als 8 C-Atome im Fettsäureteil) und natürlichen oder synthetischen Emulgatoren, wobei die Konzentrationen der wässrigen Komponenten von 0,1 bis 200 g pro Kilogramm unpolarem Kohlenwasserstoff beträgt.

Die Celluloseformkörper mit Einschlüssen aus Gemischen modifizierter Schichtsilikate, Kohlenwasserstoffen und festen oder flüssigen lipophilen Wirkstoffen bzw. W/O-Emulsionen nach diesem Verfahren können als Funktionsfaser in Mischgarnen mit anderen Natur- oder Chemiefasern beispielsweise aus Polyester, Polyamid, Polypropylen, Viskose, Baumwolle oder Wolle, in textilen Gestricken und Geweben mit funktionalem Zusatznutzen, in Funktionsvliesstoffen und Funktionsvliesstoffverbunden, in Papieren und Papierverbunden sowie in Funktionsfolien und -membranen Anwendung finden.

Die wichtigsten Methoden zur Charakterisierung der Exfolierung eines Schichtsilikat-Nanocomposites sind einerseits die Röntgenstreuung (WAXS) und andererseits die Transmissions-Elektronenmikroskopie (TEM). Beide Methoden haben allerdings nur eine bedingte Aussagekraft für eine vergleichende Beurteilung der Exfolierung von Nanocomposit-Proben. Rheologische Untersuchungen an Nanocomposit-Dispersionen bieten demgegenüber mindestens zwei wesentliche Vorteile:

i) sie fragen ein makroskopisches Probevolumen ab, benötigen aber nur wenige Gramm der Probe und
ii) sie sind physikochemische Standardmethoden und experimentell weniger aufwendig als WAXS oder TEM.

Die Methode beruht auf der Bestimmung des Scherverdünnungsexponenten n, der ein halbquantitatives Maß für die Delaminierung eines Nanoschichtsilikates ist [R. Wagener et al.:" Rheologische Charakterisierung von Nanocompositen", 8. Rudolstädter Kunststofftag, 21. Mai 2003]. Die Messungen wurden in einem Haake Mars 2 Platte-Platte-Rheometer bei kleinen Auslenkungen von weniger als 1% ausgeführt. In Voruntersuchungen wurde überprüft, dass diese Scheramplitude im Messgerät nicht zu einer ungewollten Orientierung der Plättchen führt. Die Viskosität der jeweiligen Proben wurde im Schergeschwindigkeitsbereich zwischen 0,1 und 100 Hz gemessen. An die so erhaltene Fließkurve wurde eine Potenzgleichung angepasst:

$$\eta^* = A * \omega^{(n)},$$

mit:

$\eta^*$ = experimentell ermittelte Lösungsviskosität (oder bei thermoplastischen Polymeren auch Schmelzeviskosität)
A = Vorfaktor
$\omega$ = Oszillationsfrequenz des Rheometers (äquivalent zur Schergeschwindigkeit)
n = Scherverdünnungsexponent

Aus einer doppelt logarithmischen Auftragung von $\eta^*$ gegen $\omega$ wurde der Scherverdünnungsexponent n ermittelt, indem eine Gerade an den linearen Teil des Graphen bei den niedrigsten Schergeschwindigkeiten angelegt wurde. Der Wert von n ergibt sich als Steigung der Geraden. Abbildung1 zeigt das Ergebnis einer solchen rheologischen Untersuchung an unterschiedlich stark und/oder lang gescherten und mit Schichtsilikat-Nanocompositen modifizierten Celluloselösungen.

Werte bei bzw. geringfügig unter "0" zeigen nur eine geringe Änderung der Exfolierung an. Stärker fallende Geraden sollten ein Maß für eine zunehmende Exfolierung im Schichtsilikat-Nanocomposite sein, und zeigen eine zunehmend stärkere Scherverdünnung an. Sie muss jedoch nicht zwangsläufig das Ergebnis unterschiedlicher Exfolierung in den Nanocomposites, sondern kann auch durch Temperatureffekte verursacht sein.

Deshalb wurden Proben des Composites analog Beispiel 1 einer Serie von Messungen bei unterschiedlichen Temperaturen im Intervall 85 °C < T < 115 °C unterzogen. Dabei zeigte sich im Bereich kleiner Oszillationsfrequenzen $\omega$ < 2 Hz praktisch kein Einfluss der Temperatur auf die Fließkurve. Dieses eher für Feststoffe typische Verhalten der Nanocomposites wird, wie auch bei Untersuchungen von beispielsweise Polycarbonat-Schichtsilikat-Nanocompositen gezeigt [P. Pötschke et al., "Rheological behavior of multiwalled carbon nanotube/polycarbonate composites"; Polymer 43: 2002, 3247-3255], offenbar durch eine vergleichsweise regelmäßige, räumliche Struktur von Silikatplättchen mit starken Kante/Fläche-Wechselwirkungen bestimmt.

Mit Hilfe der beschriebenen Methodik ist es gelungen, Celluloseformkörper herzustellen und halbquantitativ vergleichbar zu bewerten, bei denen die Wirkstofffreisetzung gesteuert werden kann. Zudem gelingt es auf diese Weise erstmals, auch stark flüchtige bzw. wasserlösliche Wirkstoffe in den fertigen Celluloseformkörper einzubinden und deren Freisetzung zu steuern.

*Beispiele*

Die nachfolgenden Beispiele verfolgen das Ziel, die Erfindung zu erläutern. Sie stellen mögliche Ausführungsformen des erfindungsgemäßen Verfahrens dar, ohne eine Ausschließlichkeit zu beanspruchen. Enthaltene Prozentangaben sind, soweit nicht anders angegeben, Masseprozente.

Beispiel 1

2,265 kg Cotton Linters Zellstoff (DP: 618) und 114 g Gallussäurepropylester werden mit 21,000 kg einer 60 %-igen, wässrigen NMMO-Lösung gemischt und einem Rührkessel zugeführt. Unter Rühren bei 50 min$^{-1}$ wird der Maische bei einem Vakuum von 40 mbar und einer Temperatur von 50 °C ca. 5 l Wasser entzogen. Parallel dazu werden unter 30 minütiger Scherung mittels Ultra Turrax bei 25.000 min$^{-1}$ 2,242 kg einer 80 %-igen wässrigen NMMO-Lösung und 364,5 g Schichtsilikat (Montmorillonit modifiziert mit Methyl-talg-bis-(2-hydroxyethyl)-ammonium - im Montmorillonit natürlich enthaltene Kationen sind durch diese Ammonium-Kationen ausgetauscht = Cloisite® 30 B Nanoclay von Southern Clay) dispergiert und zur Maische hinzugefügt. Der Maischeansatz wird bei 50 min$^{-1}$, 100 °C und einem Vakuum von 20 mbar solange weitergerührt bis eine hochviskose Masse entsteht. Der hochviskosen Masse wird dann eine Dispersion aus 135 g Nachtkerzenöl, 545 g n-Octadecan und 91,1 g pyrogener Kieselsäure (Aerosil® R 106), die unter starker Scherung mittels UltraTurrax separat gefertigt wurde, hinzugefügt und die gesamte Mischung bei 100 °C und 20 mbar bis zur homogenen Verteilung aller Komponenten weiter gerührt. Der Scherverdünnungsexponent wurde zu -0,86 bestimmt (Kurve d) in Abbildung 1). Nach Überführung der fertigen Spinnmasse werden mittels eines Trocken-Nass-Spinnprozesses (120 $\mu$m Düsenöffnungen, 20 mm Luftspalt) Stapelfasern mit einer Feinheit von 2,2 dtex und 60 mm Schnittlänge gefertigt.

1.500 g der so gefertigten Stapelfasern werden mit 3.500 g Baumwollfasern gemischt, einer Laborkrempel vorgelegt und nach Kreuzlegung zu einem Vlies mit einer Flächenmasse von 150 g/m$^2$ vernadelt.

Für die Messung des Wirkstoffübertritts aus der textilen Fläche auf ein technisches Hautmodell bei 25 °C und 60 % Luftfeuchte durch mechanische Beanspruchung wurde mittels eines Abriebprüfgerätes eine Tragesimulation in Anlehnung an DIN EN ISO 105-X12 2002-12 durchgeführt. Der übergetretene Wirkstoffgehalt wurde anschließend nach erschöpfender Extraktion der Haut mit Toluol mittels HPLC-MS detektiert. Der Mittelwert von 5 Parallelbestimmungen betrug 0,073 mg/100g Nachtkerzenöl.

Der bestimmte, hohe negative Scherverdünnungsexponent der Spinnlösung, der durch eine lange Scherzeit und eine hohe Schergeschwindigkeit eingestellt wurde, korreliert demnach mit einer recht langsamen Freisetzung des eingearbeiteten Nachtkerzenöls.

Beispiel 2

Einer nach Beispiel 1 hergestellten Maische wurde bei ansonsten analogem Vorgehen eine Dispersion aus 135 g Nachtkerzenöl, 545 g n-Dodecan und 91,1 g pyrogener Kieselsäure (Aerosil® R 106) zugesetzt. Die Mischung, deren Scherverdünnungsexponent n = -0,56 betrug (Kurve b) in Abbildung 1), wird danach analog Beispiel 1 weiter behandelt und verformt. Aus den erhaltenen Stapelfasern wurde ein Vlies gleicher Zusammensetzung und gleicher Flächenmasse wie in Beispiel 1 gebildet.

Bei der Messung des Wirkstoffübertritts konnte ein Mittelwert von 0,754 mg/100g Nachtkerzenöl bestimmt werden.

Durch die Veränderung der Wirkstoffmatrixzusammensetzung wird ein niedrigerer negativer Scherverdünnungsexponent bestimmt und eine schnellere Wirkstofffreisetzung erreicht.

Beispiel 3

Einer nach Beispiel 1 hergestellten Maische wurde eine parallel gefertigte Dispersion aus 2,242 kg einer 80 %-igen wässrigen NMMO-Lösung und 364,5 g Schichtsilikat (Cloisite® 30 B), die mittels UltraTurrax nur 10 Minuten dispergiert worden war, zugesetzt und analog Beispiel 1 weiterverarbeitet. Der Scherverdünnungsexponent der Lösung betrug -0,67 (Kurve c) in Abbildung 1).

Bei der Messung des Wirkstoffübertritts konnte eine mittlere Freisetzung von 0,522 mg/100g Nachtkerzenöl detektiert werden.

Die im Vergleich zu Beispiel 1 deutlich verringerte Scherdauer führt zu einem Absinken des absoluten Wertes des bestimmbaren Scherverdünnungsexponenten und bedingt eine signifikante Erhöhung der freigesetzten Wirkstoffmenge im Vergleich zu Beispiel 1.

Beispiel 4

Der nach Beispiel 1 hergestellten Maische wurde bei ansonsten analogem Vorgehen eine Dispersion aus 135 g $\alpha$-Tocopherol, 545 g Palmkernöl und 91,1 g pyrogener Kieselsäure zugesetzt. Die Mischung, deren Scherverdünnungsexponent -0,13 betrug, wird danach analog Beispiel 1 weiter behandelt und verformt. Aus den erhaltenen Stapelfasern wurde ein Vlies gleicher Zusammensetzung und gleicher Flächenmasse wie in Beispiel 1 gebildet.

Bei der Messung des Wirkstoffübertritts konnte ein Mittelwert von 1,290 mg/100g $\alpha$-Tocopherol bestimmt werden.

Im Vergleich zu den bereits dargestellten Beispielen verursacht eine Änderung der Zusammensetzung der Wirkstoffmatrix bei ansonsten vergleichbaren Parametern auch hier eine deutliche Zunahme der Freisetzungsrate.

Beispiel 5

Der nach Beispiel 1 hergestellten Maische wurde bei ansonsten analogem Vorgehen eine Dispersion aus 135 g W/O-Emulsion (Urea, Kakaobutter, Wollwachsalkohol), 545 g n-Octadecan und 91,1 g pyrogener Kieselsäure (HDK® N 20) zugesetzt. Der Scherverdünnungsexponent wurde zu -0,04 (Kurve a) in Abbildung 1) bestimmt. Die Mischung wird danach analog Beispiel 1 weiter behandelt und verformt. Aus den erhaltenen Stapelfasern wurde ein Garn aus 30 % Funktionsfasern und 70 % Baumwolle gefertigt und zu einem Feinrundstrickstück weiterverarbeitet.

Bei der Messung des Wirkstoffübertritts konnte ein Mittelwert von 2,680 mg/100g Urea bestimmt werden.

W/O-Emulsionen zeigen eine sehr sensible Beeinflussung des Scherverdünnungsexponenten von der Wirkstoffmatrixzusammensetzung bei ansonsten vergleichbaren Behandlungsparametern, die niedrigsten Scherverdünnungsexponenten und vergleichsweise hohe Freisetzungsraten.

**Patentansprüche**

1. Verfahren zur Herstellung von Celluloseformkörpern mit gesteuerter Wirkstoff-Freisetzung, mit den Stufen:

   a) Zellstoff wird in einem wässrigen Direktlösungsmittel für Cellulose zu einer Cellulosemaische dispergiert,

   b) in einem separaten Verfahrensschritt wird ein organisch modifiziertes nanoskaliges Schichtsilikat oder ein durch Ionenaustausch mit Kalium-, Calcium- oder Aluminium-Ionen voraktiviertes, nanoskaliges Schichtsilikat mit einem wässrigen Direktlösemittel für Cellulose homogenisiert und durch Scherung partiell oder vollständig exfoliert, der Cellulosemaische zugegeben und mit ihr vermischt,

   c) in einem weiteren separaten Verfahrensschritt wird eine Zusammensetzung aus einem Wirkstoff und einem lipophilen Matrixmaterial für den Wirkstoff oder eine wirkstoffhaltige Wasser-in-Öl-Emulsion durch anorganische oder organische Verdicker stabilisiert und in eine gelartige Paste überführt, diese Paste wird ebenfalls der Cellulosemaische zugegeben und unter Rühren bei Temperaturen bis 130 °C mit ihr vermischt,

   d) aus der Mischung wird Wasser abgezogen bis eine vollständige Auflösung der Cellulose erfolgt ist und

   e) die entstandene Spinnlösung wird nach einem Lösungsspinnverfahren zu Formkörpern verformt, nachbehandelt, gegebenenfalls aviviert und getrocknet.

2. Verfahren gemäß Anspruch 1, **dadurch gekennzeichnet, dass** das Direktlösemittel für Cellulose eine wässrige N-Methylmorpholin-N-oxid-Lösung, eine wasserhaltige ionische Flüssigkeit, die zusätzlich organische Lösungsmittel enthalten kann, oder eine Lösung von Dimethylacetamid (DMAc) und Lithiumchlorid ist.

3. Verfahren nach Anspruch 1, **gekennzeichnet dadurch, dass** die organisch modifizierten Schichtsilikate synthetische Schichtsilikate sind, die durch Ammoniumkationen mit mindestens einem langkettigen, unverzweigten Alkyl- und/oder Alkenylrest mit 14 oder mehr C-Atomen, bevorzugt mit 14 bis 20 C-Atomen, modifiziert sind, wobei der Alkyl- oder Alkenylrest substituiert sein kann, speziell mit einer oder mehreren Hydroxyl- oder Carboxylgruppe(n).

4. Verfahren nach einem oder mehreren der Ansprüche 1 bis 3, **dadurch gekennzeichnet, dass** der Anteil an organisch modifizierten Schichtsilikat(en) in den Celluloseformkörpern, die bevorzugt Cellulosefunktionsfasern sind, 0,5 bis 20 Gew.-%, bevorzugt 5 bis 15 Gew.-%, beträgt, jeweils bezogen auf das Gewicht der Cellulose.

5. Verfahren nach einem oder mehreren der Ansprüche 1 bis 4, **gekennzeichnet dadurch, dass** der Wirkstoff aus der Gruppe der festen oder flüssigen lipophilen Wirkstoffe ausgewählt ist, wobei kosmetische Wirkstoffe wie Nachtkerzenöl, Johanneskrautöl, Jojobaöl, Avocadoöl, fettlösliche Vitamine und Provitamine, wie Vitamin A, Retinol, Vitamin D oder Vitamin E, W/O Emulsionen oder unpolare oder wässrige Pflanzenauszüge bevorzugt sind.

6. Verfahren nach einem oder mehreren der Ansprüche 1 bis 5, **dadurch gekennzeichnet, dass** das lipophile Matrixmaterial für den Wirkstoff ein Kohlenwasserstoff mit mehr als 8 Kohlenstoffatomen, bevorzugt mit 8 bis 22 Kohlenstoffatomen, ein (C8-C22)Fettalkohol, eine (C8-C22)Fettsäure und/oder ein Fettsäureester mit 8 bis 22 Kohlenstoffatomen im Fettsäureteil ist.

7. Verfahren nach einem oder mehreren der Ansprüche 1 bis 6, **dadurch gekennzeichnet, dass** die anorganischen Verdicker Nanopartikel aus pyrogener Kieselsäure, Metalloxid-Keramik und/oder Metall sind.

8. Verfahren nach einem oder mehreren der Ansprüche 1 bis 7, **dadurch gekennzeichnet, dass** die organischen Verdicker aliphatisch-aromatische Blockcopolymere sind.

9. Verfahren nach einem oder mehreren der Ansprüche 1 bis 8, **dadurch gekennzeichnet, dass** die Zusammensetzung aus Wirkstoff und lipophilem Material in Konzentrationen von 0,1 bis 200 g pro Kilogramm Cellulose eingesetzt wird.

10. Verfahren nach Anspruch 9, **dadurch gekennzeichnet, dass** die Wasser-in-Öl-Emulsion eine in einer öligen Phase dispergierte hydrophile Phase, bevorzugt eine wässrige Zubereitung von Kosmetikwirkstoffen oder wässrigen Auszügen von Pflanzeninhaltsstoffen, jeweils gemischt mit unpolaren Kohlenwasserstoffen, Fettalkoholen, Fettsäuren und Fettsäureestern mit mehr als 8 Kohlenstoffatomen und natürlichen oder synthetischen Emulgatoren umfasst, wobei die Konzentrationen der wässrigen Komponenten von 0,1 bis 200 g pro Kilogramm flüssiger Phase beträgt.

11. Verfahren nach einem oder mehreren der Ansprüche 1 bis 10, **dadurch gekennzeichnet, dass** die Freisetzung der Wirkstoffe gesteuert wird durch den Grad der Exfolierung der Schichtsilikate, durch die chemische Struktur und die Konzentration der organischen Kationen im Schichtsilikat, durch die Größe der interkalierten Ionen oder den Quellgrad nicht organisch modifizierter Schichtsilikate, durch die Temperatur beim Vorquellen des organisch modifizierten Schichtsilikats und/oder bei der Herstellung der Paste aus Wirkstoff und lipophilem Material für den Wirkstoff, durch die Viskosität des dabei verwendeten Dispergiermittels, durch den Wasseranteil darin, durch die Art des lipophilen Matrixmaterials sowie die Intensität und Dauer der Mischung/Scherung der organisch modifizierten Schichtsilikate.

12. Verfahren nach einem oder mehreren der Ansprüche 1 bis 11, **gekennzeichnet dadurch, dass** die anorganischen Nanopartikel in einem Anteil von 0,1 bis 10 Gew.-%, bezogen auf das Gesamtgewicht der Mischung aus Wirkstoff und lipophilem Material für den Wirkstoff, eingesetzt werden.

13. Verfahren gemäß einem oder mehreren der Ansprüche 1 bis 12, **dadurch gekennzeichnet, dass** der Scherverdünnungsexponent n der Spinnlösung im Bereich von 0,0 bis -1,2, bevorzugt im Bereich von -0,1 bis -1,0, liegt.

14. Celluloseformkörper mit organisch modifizierten nanoskaligen Schichtsilikaten oder durch Ionenaustausch mit Kalium-, Calcium- oder Aluminium-Ionen voraktivierten, nanoskaligen Schichtsilikaten und darin fein verteilten Domänen aus Zusammensetzungen von Wirkstoffen und lipophilen Matrixmaterialien für die Wirkstoffe oder wirkstoffhaltigen Wasser-in-Öl-Emulsionen, hergestellt nach einem Verfahren nach den Ansprüchen 1 bis 13, **dadurch**

**gekennzeichnet, dass** die organisch modifizierten Schichtsilikate modifiziert sind durch Ammonium- oder PhosphoniumKationen mit mindestens einem geradkettigen Kohlenwasserstoffrest, der 14 oder mehr C-Atome, bevorzugt 14 - 20 C-Atome, aufweist.

15. Celluloseformkörper nach Anspruch 14, **dadurch gekennzeichnet, dass** sie in textilen Anwendungen eingesetzt werden und mit hoch flüchtigen, thermo- und/oder chemosensiblen Wirkstoffen wieder beladbar sind.

16. Verwendung der Celluloseformkörper nach Anspruch 14 oder 15 als Funktionsfaser in Mischgarnen mit Polyesterfasern, Polyamidfasern, Polypropylenfasern, Viskosefasern, Baumwollfasern oder Wolle, in textilen Gestricken und Geweben, in Vliesstoffen und Vliesstoffverbunden, in Papier und Papierverbunden sowie in Folien und Membranen.

## Claims

1. Process for producing cellulose shaped bodies with controlled active ingredient release, comprising the steps:

   a) cellulose is dispersed in an aqueous direct solvent for cellulose to afford a cellulose mash,
   b) in a separate process step, an organically modified nanoscale phyllosilicate or a nanoscale phyllosilicate pre-activated by ion exchange with potassium, calcium or aluminium ions is homogenized with an aqueous direct solvent for cellulose and partially or completely exfoliated by shearing, added to the cellulose mash and mixed therewith,
   c) in a further separate process step, a composition of an active ingredient and a lipophilic matrix material for the active ingredient or an active ingredient-containing water-in-oil emulsion is stabilized using inorganic or organic thickeners and converted into a gel-like paste, this paste is likewise added to the cellulose mash and mixed therewith with stirring at temperatures up to 130°C,
   d) water is withdrawn from the mixture until complete dissolution of the cellulose has occurred and
   e) the resulting spinning solution is formed into shaped bodies by a solution spinning process, subjected to aftertreatment, optionally finishing and dried.

2. Process according to Claim 1, **characterized in that** the direct solvent for cellulose is an aqueous N-methylmorpholine-N-oxide solution, a water-containing ionic liquid, which may additionally contain organic solvents, or a solution of dimethylacetamide (DMAc) and lithium chloride.

3. Process according to Claim 1, **characterized in that** the organically modified phyllosilicates are synthetic phyllosilicates modified by ammonium cations having at least one long-chain unbranched alkyl and/or alkenyl radical having 14 or more C atoms, preferably having 14 to 20 C atoms, wherein the alkyl or alkenyl radical may be substituted, especially with one or more hydroxyl or carboxyl group(s).

4. Process according to one or more of Claims 1 to 3, **characterized in that** the proportion of organically modified phyllosilicate(s) in the cellulose shaped bodies, which are preferably cellulose functional fibres, is 0.5% to 20% by weight, preferably 5% to 15% by weight, in each case based on the weight of the cellulose.

5. Process according to one or more of Claims 1 to 4, **characterized in that** the active ingredient is selected from the group of solid or liquid lipophilic active ingredients, wherein preference is given to cosmetic active ingredients such as evening primrose oil, St John's wort oil, jojoba oil, avocado oil, fat-soluble vitamins and provitamins, such as vitamin A, retinol, vitamin D or vitamin E, W/O emulsions or nonpolar or aqueous plant extracts.

6. Process according to one or more of Claims 1 to 5, **characterized in that** the lipophilic matrix material for the active ingredient is a hydrocarbon having more than 8 carbon atoms, preferably having 8 to 22 carbon atoms, a (C8-C22) fatty alcohol, a (C8-C22) fatty acid and/or a fatty acid ester having 8 to 22 carbon atoms in the fatty acid portion.

7. Process according to one or more of Claims 1 to 6, **characterized in that** the inorganic thickeners are nanoparticles of pyrogenic silica, metal oxide ceramic and/or metal.

8. Process according to one or more of Claims 1 to 7, **characterized in that** the organic thickeners are aliphatic-aromatic block copolymers.

9. Process according to one or more of Claims 1 to 8, **characterized in that** the composition of active ingredient and lipophilic material is employed in concentrations of 0.1 to 200 g per kilogram of cellulose.

10. Process according to Claim 9, **characterized in that** the water-in-oil emulsion comprises a hydrophilic phase dispersed in an oily phase, preferably an aqueous preparation of cosmetic active ingredients or aqueous extracts of plant ingredients, in each case mixed with nonpolar hydrocarbons, fatty alcohols, fatty acids and fatty acid esters having more than 8 carbon atoms and natural or synthetic emulsifiers, wherein the concentration of the aqueous components is from 0.1 to 200 g per kilogram of liquid phase.

11. Process according to one or more of Claims 1 to 10, **characterized in that** the release of the active ingredients is controlled by the extent of the exfoliation of the phyllosilicates, by the chemical structure and the concentration of the organic cations in the phyllosilicate, by the size of the intercalated ions or the degree of swelling of organically unmodified phyllosilicates, by the temperature during the pre-swelling of the organically modified phyllosilicate and/or during the production of the paste from active ingredient and lipophilic material for the active ingredient, by the viscosity of the dispersant used in the process, by the water content therein, by the type of the lipophilic matrix material and the intensity and duration of the mixing/shearing of the organically modified phyllosilicates.

12. Process according to one or more of Claims 1 to 11, **characterized in that** the inorganic nanoparticles are employed in a proportion of 0.1% to 10% by weight, based on the total weight of the mixture of active ingredient and lipophilic material for the active ingredient.

13. Process according to one or more of Claims 1 to 12, **characterized in that** the shear thinning exponent n of the spinning solution is in the range from 0.0 to -1.2, preferably in the range from -0.1 to -1.0.

14. Cellulose shaped bodies comprising organically modified nanoscale phyllosilicates or nanoscale phyllosilicates pre-activated by ion exchange with potassium, calcium or aluminium ions and regions, finely dispersed therein, of compositions of active ingredients and lipophilic matrix materials for the active ingredients or active ingredient-containing water-in-oil emulsions, produced by a process according to Claims 1 to 13, **characterized in that** the organically modified phyllosilicates are modified by ammonium or phosphonium cations having at least one straight-chain hydrocarbon radical comprising 14 or more C atoms, preferably 14-20 C atoms.

15. Cellulose shaped bodies according to Claim 14, **characterized in that** they are employed in textile applications and are reloadable with highly volatile, thermally and/or chemically sensitive active ingredients.

16. Use of the cellulose shaped bodies according to Claim 14 or 15 as a functional fibre in hybrid yarns with polyester fibres, polyamide fibres, polypropylene fibres, viscose fibres, cotton fibres or wool, in textile knits and wovens, in nonwovens and nonwoven laminates, in paper and paper laminates and in films and membranes.

## Revendications

1. Procédé de fabrication de corps façonnés en cellulose pourvus d'une libération régulée de substance active, comprenant les étapes :

   a) la cellulose est dispersée dans un solvant aqueux direct pour la cellulose en un moût de cellulose,
   b) dans une étape de procédé séparée, un silicate en couches nanométrique, organiquement modifié ou un silicate en couches nanométrique, préactivé par un échange ionique avec des ions de potassium, de calcium ou d'aluminium est homogénéisé avec un solvant direct aqueux pour la cellulose et exfolié partiellement ou complètement par cisaillement, ajouté au moût de cellulose et mélangé avec celui-ci,
   c) dans une autre étape de procédé séparée, une composition constituée par une substance active et une matière de matrice lipophile pour la substance active ou une émulsion eau-dans-huile contenant une substance active est stabilisée par des épaississants inorganiques ou organiques et transformée en une pâte de type gel, cette pâte est également ajoutée au moût de cellulose et mélangée avec celui-ci sous agitation à des températures jusqu'à 130°C,
   d) l'eau est soutirée du mélange jusqu'à ce qu'une dissolution complète de la cellulose ait eu lieu et
   e) après un procédé de filage en solution, la solution de filage formée est façonnée en corps façonnés, post-traitée, le cas échéant activée et séchée.

2. Procédé selon la revendication 1, **caractérisé en ce que** le solvant direct pour la cellulose est une solution aqueuse de N-oxyde de N-méthylmorpholine, un liquide ionique contenant de l'eau, qui peut contenir en plus du solvant organique, ou une solution de diméthylacétamide (DMAc) et de chlorure de lithium.

3. Procédé selon la revendication 1, **caractérisé en ce que** les silicates en couches organiquement modifiés sont des silicates en couches synthétiques, qui sont modifiés par des cations d'ammonium présentant au moins un radical alkyle et/ou alcényle à longue chaîne, non ramifié, comprenant 14 atomes de carbone ou plus, de préférence 14 à 20 atomes de carbone, le radical alkyle ou alcényle pouvant être substitué, en particulier par un ou plusieurs groupes hydroxyle ou carboxyle.

4. Procédé selon l'une ou plusieurs des revendications 1 à 3, **caractérisé en ce que** la proportion de silicate(s) en couches organiquement modifié(s) dans les corps façonnés de cellulose, qui sont de préférence des fibres fonctionnelles de cellulose, représente 0,5 à 20% en poids, de préférence 5 à 15% en poids, à chaque fois par rapport au poids de la cellulose.

5. Procédé selon l'une ou plusieurs des revendications 1 à 4, **caractérisé en ce que** la substance active est choisie dans le groupe des substances actives lipophiles solides ou liquides, des substances actives cosmétiques, telles que l'huile d'onagre, l'huile de millepertuis, l'huile de jojoba, l'huile d'avocat, les vitamines et les provitamines solubles dans la graisse, telles que la vitamine A, le rétinol, la vitamine D ou la vitamine E, des émulsions E/H ou des extraits végétaux non polaires ou aqueux, étant préférées.

6. Procédé selon l'une ou plusieurs des revendications 1 à 5, **caractérisé en ce que** la matière de matrice lipophile pour la substance active est un hydrocarbure comprenant plus de 8 atomes de carbone, de préférence 8 à 22 atomes de carbone, un alcool gras en C8-C22, un acide gras en C8-C22 et/ou un ester d'acide gras comprenant 8 à 22 atomes de carbone dans la partie acide gras.

7. Procédé selon l'une ou plusieurs des revendications 1 à 6, **caractérisé en ce que** les épaississants inorganiques sont des nanoparticules en silice pyrogène, en céramique d'oxyde métallique et/ou en métal.

8. Procédé selon l'une ou plusieurs des revendications 1 à 7, **caractérisé en ce que** les épaississants organiques sont des copolymères séquencés aliphatiques-aromatiques.

9. Procédé selon l'une ou plusieurs des revendications 1 à 8, **caractérisé en ce que** la composition de substance active et de matière lipophile est utilisée en des concentrations de 0,1 à 200 g par kilogramme de cellulose.

10. Procédé selon la revendication 9, **caractérisé en ce que** l'émulsion eau-dans-huile comprend une phase hydrophile dispersée dans une phase huileuse, de préférence une préparation aqueuse de substances actives cosmétiques ou d'extraits aqueux de constituants végétaux, à chaque fois mélangée avec des hydrocarbures non polaires, des alcools gras, des acides gras et des esters d'acide gras comprenant plus de 8 atomes de carbone et des émulsifiants naturels ou synthétiques, la concentration en composants aqueux représentant 0,1 à 200 g par kilogramme de phase liquide.

11. Procédé selon l'une ou plusieurs des revendications 1 à 10, **caractérisé en ce que** la libération des substances actives est régulée par le taux d'exfoliation des silicates en couches, par la structure chimique et la concentration en cations organiques dans le silicate en couches, par la grosseur des ions intercalés ou le degré de gonflement de silicates en couches non organiquement modifiés, par la température lors du prégonflement du silicate en couches organiquement modifié et/ou lors de la préparation de la pâte constituée par la substance active et la matière lipophile pour la substance active, par la viscosité du dispersant utilisé lors de ladite préparation, par la proportion d'eau dans ledit dispersant, par le type de matière de matrice lipophile ainsi que l'intensité et la durée du mélange/cisaillement des silicates en couches organiquement modifiés.

12. Procédé selon l'une ou plusieurs des revendications 1 à 11, **caractérisé en ce que** les nanoparticules inorganiques sont utilisées en une proportion de 0,1 à 10% en poids, par rapport au poids total du mélange de la substance active et de la matière lipophile pour la substance active.

13. Procédé selon l'une ou plusieurs des revendications 1 à 12, **caractérisé en ce que** l'exposant de dilution par cisaillement n de la solution de filage se situe dans la plage de 0,0 à -1,2, de préférence dans la plage de -0,1 à -1,0.

14. Corps façonnés en cellulose présentant des silicates en couches nanométriques, organiquement modifiés ou des silicates en couches nanométriques, préactivés par échange ionique avec des ions de potassium, de calcium ou d'aluminium, et des domaines finement répartis dans ceux-ci de compositions de substances actives et de matières de matrice lipophiles pour les substances actives ou des émulsions eau-dans-huile contenant des substances actives, préparés selon un procédé selon les revendications 1 à 13, **caractérisés en ce que** les silicates en couches organiquement modifiés sont modifiés par des cations d'ammonium ou de phosphonium comprenant au moins un radical hydrocarboné linéaire qui présente 14 atomes de carbone ou plus, de préférence 14-20 atomes de carbone.

15. Corps façonnés en cellulose selon la revendication 14, **caractérisés en ce qu'**ils sont utilisés dans des applications textiles et peuvent être rechargés par des substances actives hautement volatiles, thermosensibles et/ou sensibles aux produits chimiques.

16. Utilisation des corps façonnés en cellulose selon la revendication 14 ou 15 en tant que fibres fonctionnelles dans des fils mixtes avec des fibres de polyester, des fibres de polyamide, des fibres de polypropylène, des fibres de viscose, des fibres de coton ou de la laine, dans des tricots et des tissus textiles, dans des non-tissés et des composites à base de non-tissés, dans du papier et des composites à base de papier ainsi que dans des feuilles et des membranes.

Abbildung 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009062657 A
- DE 102006046358 A1
- DE 102010007497 A1
- US 7244497 B2
- WO 2005017247 A2
- DE 202004021259 U1
- US 5153066 A
- DE 69600181 T2
- DE 102004014704 A1
- DE 102007054702 A1

**In der Beschreibung aufgeführte Nicht-Patentliteratur**

- **T. FORNES** ; **P. YOON** ; **D. HUTTER** ; **H. KESKKU-LA** ; **D. PAU**. *Polymer*, 2002, vol. 43, 5915
- **T. FORNES** ; **D. HUTTER** ; **D. PAUL**. *Macromolecules*, 2004, vol. 37, 1793
- **N. HASEGAWA** ; **H. OKAMOTO** ; **M. KATO** ; **A. USUKI** ; **N. SATO**. *Polymer*, 2003, vol. 44, 2933
- **N. FEDULLO** ; **M. SCLAVONS** ; **C. BAILLY** ; **J.-M. LEFEBVRE** ; **J. DEVAUX**. *Macromol. Symp.*, 2006, vol. 233, 235
- **R. WAGENER et al.** Rheologische Charakterisierung von Nanocompositen. *Rudolstädter Kunststoff tag*, 21 May 2003, vol. 8
- **P. PÖTSCHKE et al.** Rheological behavior of multiwalled carbon nanotube/polycarbonate composites. *Polymer*, 2002, vol. 43, 3247-3255